# EUROPÄISCHE PATENTANMELDUNG

(11) **EP 2 201 928 A2**
(43) Veröffentlichungstag der Anmeldung: **30.06.2010**
(21) Anmeldenummer: 09180538.2
(22) Anmeldetag: 23.12.2009
(51) Int. Cl.: A61K 8/06, A61K 8/34, A61K 8/49, A61K 8/60, A61K 8/895, A61Q 17/04

(54) **Sonnenschutzzusammensetzungen**

(30) Priorität: 29.12.2008 DE 102008063306
(71) Anmelder: Henkel AG & Co. KGaA, 40589 Düsseldorf (DE)
(72) Erfinder: Janßen, Frank, 41470, Neuss (DE); Dickhof, Susanne, 41748, Viersen (DE)

(57) **Zusammenfassung**

Gegenstand der vorliegenden Anmeldung sind kosmetische Zusammensetzungen mit verbesserter Lagerstabilität des Lichtschutzfaktors, enthaltend mindestens einen linearen, gesättigten primären Alkohol mit 14 - 30 Kohlenstoffatomen, mindestens ein Alkylglycosid mit einem linearen oder verzweigten Alkyl- oder Alkenylrest mit 14 - 30 Kohlenstoffatomen und einem Mono-, Di- oder Triglycosidrest, Polysilicone-15 und mindestens einen wasserlöslichen UV-Filter, umfassend ein Derivat der Benzimidazolsulfonsäure, die einen pH-Wert bei 20°C von 6,0 - 8,0, aufweisen.

## Beschreibung

Die vorliegende Erfindung betrifft kosmetische und dermatologische Zusammensetzungen zum Schutz von Haut und Haar gegen UV-Strahlung.

Die hautschädigende Wirkung des ultravioletten Teils der Sonnenstrahlung ist bekannt. Die UV-Strahlung im Bereich zwischen 290 nm und 320 nm, dem so genannten UV-B-Bereich, kann zu einem Erythem, einem einfachen Sonnenbrand oder sogar mehr oder weniger starken Verbrennungen als akuten Erscheinungsformen führen. Als ein Maximum der Erythemwirksamkeit des Sonnenlichtes wird der engere Bereich um 308 nm angegeben. Man hat lange Zeit fälschlicherweise angenommen, dass die längerwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, dass UV-A-Strahlung im Hinblick auf die Auslösung photodynamischer, speziell phototoxischer Reaktionen und chronischer Veränderungen der Haut, weitaus gefährlicher als UV-B-Strahlung ist. Auch kann der schädigende Einfluss der UV-B-Strahlung durch UV-A-Strahlung noch verstärkt werden. So ist es u. a. erwiesen, dass selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhans-Zellen und eine leichte, chronische Entzündung aus. Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden. Es ist daher von grundsätzlicher Wichtigkeit, dass kosmetische und dermatologische Lichtschutzzusammensetzungen sowohl gegen UV-B- als auch gegen UV-A-Strahlung ausreichenden Schutz bieten. Hierzu sind im Stand der Technik schon eine Vielzahl von kosmetischen Zusammensetzungen entwickelt worden, die mindestens eine UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UV-A-Filter und UV-B-Filter, je nachdem, in welchem Wellenlängenbereich das Absorptionsmaximum des Filters liegt.

Organische UV-Filter in den für einen hohen Lichtschutzfaktor notwendigen Mengen lassen sich häufig nur mit großen Schwierigkeiten so in kosmetische Träger einarbeiten, dass langzeitstabile Zusammensetzungen mit angenehmen, insbesondere nicht-klebrigen Eigenschaften, erhalten werden.

Zur Herstellung besonders leistungsstarker Lichtschutzzusammensetzungen auf Basis von Öl-Wasser-Emulsionen (O/W oder W/O) ist es vorteilhaft, wenn sowohl in der Ölphase als auch in der Wasserphase mindestens ein organischer UV-Filter enthalten ist. Da UV-Filter in großen Wirtschaftsräumen, wie den USA, der EU, Japan oder Australien, zulassungspflichtige Substanzen darstellen, ist der Kosmetikfachmann bei der Herstellung neuer Lichtschutzzusammensetzungen an eine relativ begrenzte Palette zugelassener UV-Filter gebunden. Ein besonders bevorzugter wasserlöslicher organischer UV-Filter hieraus ist 2-Phenylbenzimidazol-5-sulfonsäure. Diese Verbindung neigt allerdings dazu, bei längerer Lagerung auszukristallieren, was den SPF (Sun Protection Factor, Lichtschutzfaktor) der gesamten Zusammensetzung drastisch absinken lässt.

### Aufgaben

Die Zusammensetzungen des Standes der Technik sind weiter verbesserbar. Insbesondere weisen Zusammensetzungen, die Kombinationen von UV-Filtersubstanzen oder anderen Wirkstoffen enthalten, bisweilen in Bezug auf die Einsatzkonzentrationen formulierungstechnische Schwierigkeiten auf, die das Erreichen guter UV-Schutzleistung erschweren. Ein weiterer Nachteil des Standes der Technik ist, dass übliche Lichtschutzformulierungen einen meist klebrigen Film auf der Haut hinterlassen.

Aufgabe der vorliegenden Erfindung war es, Lichtschutzzusammensetzungen bereitzustellen, die einen hohen Schutz gegen die oben genannten verschiedenen nachteiligen Wirkungen der Strahlung im UV-A-und UV-B-Bereich, insbesondere Sonnenstrahlung, bieten und gleichzeitig verbesserte sensorische Eigenschaften aufweisen. Die erfindungsgemäßen Zusammensetzungen sollen ferner gute hautpflegende Eigenschaften, insbesondere Wirksamkeit gegen Hautalterungserscheinungen (z.B. Faltenbildung, Elastizitätsverlust), sowie ein angenehmes Hautgefühl und eine leichte Verteilbarkeit auf der Haut aufweisen. Weiterhin sollten die erfindungsgemäßen Zusammensetzungen mit einer möglichst geringen Konzentration an organischen UV-Filtern einen möglichst hohen Lichtschutzfaktor (SPF) erzielen. Besonders bevorzugt sollte ein SPF von mindestens 30, bevorzugt von mindestens 40, erzielt werden.

Weiterhin sollten Öl-in-Wasser-Emulsionen mit Lichtschutzfaktor bereitgestellt werden, die ein stabiles und dennoch hautverträgliches Emulgatorsystem umfassen. Weiterhin sollten erfindungsgemäße Zusammensetzungen mit einem möglichst langzeit- und lagerstabilen SPF bereitgestellt werden, deren anfänglich gemessener SPF nach mehrwöchiger Lagerung möglichst wenig abnimmt. Eine weitere Aufgabe der vorliegenden Erfindung war es, Lichtschutzzusammensetzungen, die mindestens einen wasserlöslichen organischen UV-Filter enthalten, in lagerstabiler Form bereitzustellen.

All diese Aufgaben werden in überraschender Weise vom Gegenstand der Ansprüche der vorliegenden Anmeldung gelöst.

Gegenstand der vorliegenden Anmeldung sind kosmetische Öl-in-Wasser-Emulsionen, enthaltend
a) mindestens einen linearen, gesättigten primären Alkohol mit 14 - 30 Kohlenstoffatomen,
b) mindestens ein Alkylglycosid mit einem linearen oder verzweigten Alkyl- oder Alkenylrest mit 14 - 30 Kohlenstoffatomen und einem Mono-, Di- oder Triglycosidrest,
c) Polysilicone-15,
d) mindestens einen wasserlöslichen UV-Filter, umfassend ein Derivat der Benzimidazolsulfonsäure,
die einen pH-Wert bei 20°C von 6,0 - 8,0, aufweisen.

Bevorzugte erfindungsgemäße kosmetische Öl-in-Wasser-Emulsionen weisen bei 20°C einen pH-Wert im Bereich von 6,5 - 7,5, besonders bevorzugt einen pH-Wert im Bereich von 6,8 bis 7,3 auf.

Überraschend wurde festgestellt, dass mit einem pH-Wert im lediglich schwach sauren bis neutralen oder sogar leicht basischen Bereich eine deutlich verbesserte Langzeitstabilität des anfänglichen SPF erzielt wird.

Die Begriffe "erfindungsgemäße Zusammensetzung" und "erfindungsgemäße Öl-in-Wasser-Emulsion" werden synonym gebraucht.

Als eine Hauptkomponente enthalten die erfindungsgemäßen Zusammensetzungen mindestens einen linearen, gesättigten primären Alkohol mit 14 - 30 Kohlenstoffatomen.

Erfindungsgemäß bevorzugte lineare, gesättigte primäre Alkohole mit 14 - 30 Kohlenstoffatomen sind ausgewählt aus Myristylalkohol, Cetylalkohol, Stearylalkohol, 12-Hydroxystearylalkohol, Arachidylalkohol (Arachylalkohol), Behenylalkohol, Lignocerylalkohol, Cerylalkohol und Myricylalkohol und Mischungen hiervon, insbesondere technische Mischungen wie Arachidylalkohol und

Behenylalkohol sowie Cetylalkohol und Stearylalkohol (Cetearylalkohol). Besonders bevorzugt sind Cetylalkohol, Stearylalkohol, Arachidylalkohol und Behenylalkohol und Mischungen hiervon. Besonders bevorzugt sind Mischungen aus Cetearylalkohol und Behenylalkohol.

Die vorgenannten Alkohole werden im Folgenden auch synonym als "Fettalkohole" bezeichnet.

Bevorzugte erfindungsgemäße Öl-in-Wasser-Emulsionen enthalten einen Teil des gesamten Fettalkoholgehaltes als Teil einer Öl-in-Wasser-Emulgatormischung mit dem ebenfalls obligatorischen Alkylglycosid b) gemäß Anspruch 1, den anderen Teil des gesamten Fettalkoholgehaltes als zusätzlichen Stabilisator. Die C-Kettenlänge der Fettalkohole, die Teil der Öl-in-Wasser-Emulgatormischung mit dem Alkylglycosid sind, stimmt bevorzugt mit der C-Kettenlänge des Alkylrests des Alkylglycosids überein. Die C-Kettenlänge der Fettalkohole des anderen Teils kann davon abweichen. Besonders bevorzugt sind Mischungen aus Cetearylalkohol, der aus der Öl-in-Wasser-Emulgatormischung mit dem Cetearylglycosid stammt, und Behenylalkohol.

Besonders bevorzugte erfindungsgemäße Öl-in-Wasser-Emulsionen sind **dadurch gekennzeichnet, dass** sie mindestens einen linearen, gesättigten primären Alkohol mit 14 - 30 Kohlenstoffatomen in einer Gesamtmenge von 0,5 - 8 Gew.-%, bevorzugt 1 - 7 Gew.-%, besonders bevorzugt 1,5 - 6 Gew.-%, außerordentlich bevorzugt 2 - 5,5 Gew.-% enthalten, jeweils bezogen auf das Gesamtgewicht der Komponente in der gesamten Zusammensetzung. Auch Gesamtmengen von 2,5, 3, 3,5, 4, 4,5 und 5 Gew.-% mindestens eines linearen, gesättigten primären Alkohols mit 14 - 30 Kohlenstoffatomen können bevorzugt sein.

Als eine weitere Hauptkomponente enthalten die erfindungsgemäßen Zusammensetzungen mindestens ein Alkylglycosid mit einem linearen oder verzweigten Alkyl- oder Alkenylrest mit 14 - 30 Kohlenstoffatomen und einem Mono-, Di- oder Triglycosidrest.

Die auch als Zuckertenside vom Typ der Alkyl- und Alkenyloligoglycoside bezeichneten Komponenten weisen die allgemeine Formel (E4-II) auf,

R⁴O-[G]ₚ (E4-II)

in der R⁴ für einen Alkyl- oder Alkenylrest mit 14 - 30, bevorzugt 16 - 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglycoside können sich von Aldosen bzw. Ketosen mit 5 oder 6 Kohlenstoffatomen, wie insbesondere Fructose, Glucose und Xylose, vorzugsweise von Glucose (so genannte APG) oder Xylose (so genannte APX), ableiten, wobei Glucose als Zuckerrest außerordentlich bevorzugt ist. Die bevorzugten Alkyl- und/oder Alkenyloligoglycoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglycosiden, an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkyloligoglycosid eine analytisch ermittelte rechnerische Größe, die meistens eine gebrochene Zahl darstellt. Vorzugsweise werden Alkyl- und/oder Alkenyloligoglycoside mit einem mittleren Oligomerisierungsgrad p von 1,1 bis 3,0 eingesetzt. Aus anwendungstechnischer Sicht sind solche Alkyl- und/oder Alkenyloligoglycoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 14 - 30, bevorzugt 16 - 22 Kohlenstoffatomen ableiten. Typische Beispiele sind Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische.

Besonders bevorzugt werden die Alkyl- und Alkenyloligoglycoside als Gemisch mit dem entsprechenden, zur Alkylierung eingesetzten Alkoholschnitt eingesetzt. Hierbei handelt es sich um so genannte selbstemulgierende Mischungen aus langkettigen Fettalkoholen und entsprechend alkylierten Glycosiden. Bevorzugt werden Mischungen eingesetzt, die unter den Bezeichnungen Montanov 68, Montanov 202, Montanov L und Montanov 82 von der Firma Seppic erhältlich sind. Diese Mischungen enthalten ca. 15 Gew.-% Alkylglucosid und ca. 85 Gew.-% Fettalkohole. Selbstverständlich können die Alkyl- und Alkenyloligoglycoside aber auch in reiner Form eingesetzt werden, z. B. das Cetearyl Glucoside, erhältlich beispielsweise unter der Bezeichnung Tego Care CG 90. Bevorzugte derartige Mischungen sind (gemäß der INCI-Bezeichnung): C14-22 Alcohols, C12-20 Alkyl Glucoside (Montanov L), Arachidyl Alcohol, Behenyl Alcohol, Arachidyl Glucoside (Montanov 202), Cetearyl Alcohol, Cetearyl Glucoside (Montanov 68, Emulgade PL 68/50), Cetearyl Alcohol, Coco-Glucoside (Montanov 82).

Besonders bevorzugte erfindungsgemäße Öl-in-Wasser-Emulsionen sind **dadurch gekennzeichnet, dass** sie mindestens ein Alkylglycosid mit einem linearen oder verzweigten Alkyl- oder Alkenylrest mit 14 - 30 Kohlenstoffatomen und einem Mono-, Di- oder Triglycosidrest in einer Gesamtmenge von 0,1 - 5 Gew.-%, bevorzugt 0,3 - 4 Gew.-%, besonders bevorzugt 0,4 - 2 Gew.-%, außerordentlich bevorzugt 0,5 - 1,5 Gew.-% enthalten, jeweils bezogen auf das Gesamtgewicht der Komponente in der gesamten Zusammensetzung. Auch Gesamtmengen von 0,6, 0,7, 0,8, 0,9, 1, 1,1, 1,2, 1,3 und 1,4 Gew.-% können besonders bevorzugt sein.

Weitere besonders bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest in einer Gesamtmenge von 0,5 Gew.-% bis 8 Gew.-%, besonders bevorzugt 1 bis 6 Gew.-%, insbesondere 2,5 - 4,5 Gew.-% und außerordentlich bevorzugt 3 - 4 Gew.-%, enthalten.

Weitere besonders bevorzugte erfindungsgemäße Öl-in-Wasser-Emulsionen sind **dadurch gekennzeichnet, dass** sie ein Gemisch aus Cetearyl Alcohol und Cetearyl Glucoside in einer Gesamtmenge von 0,5 Gew.-% bis 8 Gew.-%, besonders bevorzugt 1 bis 6 Gew.-%, insbesondere 2,5 - 4,5 Gew.-% und außerordentlich bevorzugt 3 - 4 Gew.-%, enthalten.

Eine Stabilisierung des Lichtschutzfaktors (SPF) im vorstehend angegebenen pH-Bereich in Kombination mit dem mindestens einen linearen, gesättigten primären Alkohol mit 14 - 30 Kohlenstoffatomen und dem mindestens einen Alkylglycosid mit einem linearen oder verzweigten Alkyl- oder Alkenylrest mit 14 - 30 Kohlenstoffatomen und einem Mono-, Di- oder Triglycosidrest konnte insbesondere in Gegenwart des öllöslichen organischen UV-Filters Polysilicone-15 beobachtet werden.

Daher enthalten die erfindungsgemäßen kosmetischen Öl-in-Wasser-Emulsionen Polysilicone-15. Die Substanz mit der INCI-Bezeichnung Polysilicone-15 wird auch als 3-(4-(2,2-Bis-Ethoxycarbonylvinyl)-phenoxy)propenyl)-methoxysiloxan/Dimethylsiloxan-Copolymer mit der Parsol^{®} SLX), Dimethicodiethylbenzalmalonat, Diethylbenzylidene Malonate Dimethicone oder Diethylmalonylbenzylidene Oxypropene Dimethicone bezeichnet und ist unter dem Handelsnamen Parsol® SLX (INCI-Bezeichnung Dimethicodiethylbenzal malonate (CAS-Nr. 207574-74-1)) von DSM erhältlich. Die chemische Struktur ist beispielsweise in EP 709080 A2 beschrieben. Polysilicone-15 ist bevorzugt in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, enthalten, jeweils bezogen auf das Gesamtgewicht der erfindungsgemäßen Zusammensetzung.

Aufgrund des angenehmeren Hautgefühls bevorzugen viele Verbraucher Öl-in-Wasser-Emulsionen gegenüber Wasser-in-Öl-Emulsionen. Da die äußere Wasserphase in solchen Emulsionen häufig einen höheren Gewichtsanteil hat als die dispergierte Öl-/Fettphase, ist es vorteilhaft, die UV-Filtersubstanzen auf beide Phasen zu verteilen und also neben der mindestens einen öllöslichen auch mindestens eine wasserlösliche UV-Filtersubstanz einzuarbeiten.

Daher enthalten die erfindungsgemäßen kosmetischen Öl-in-Wasser-Emulsionen mindestens einen wasserlöslichen organischen UV-Filter, der mindestens ein Derivat der Benzimidazolsulfonsäure umfasst.

Bevorzugte erfindungsgemäße kosmetische Öl-in-Wasser-Emulsionen sind **dadurch gekennzeichnet, dass** mindestens ein wasserlöslicher organischer UV-Filter, ausgewählt aus mindestens einem Derivat der Benzimidazolsulfonsäure, in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, enthalten ist, jeweils bezogen auf das Gesamtgewicht der erfindungsgemäßen Zusammensetzung.

### Derivat der Benzimidazolsulfonsäure

Erfindungsgemäß bevorzugte Derivate der Benzimidazolsulfonsäure als wasserlösliche organische UV-Filtersubstanz(en) ist bzw. sind die Phenylen-1,4-bis-(2-benzimidazyl)-3,3'-5,5'-tetrasulfonsäure (UV-A) und ihre Salze, insbesondere die Alkali-, Erdalkali-, Ammonium-, Alkylammonium-, Alkanolamin- und Glucammoniumsalze, bevorzugt die entsprechenden Natrium-, Kalium-, Trialkylammonium- oder Triethanolamin-Salze. Ebenfalls bevorzugt sind die Salze dieser Säure mit Amino-C₁-C₆-Alkanolen und Amino-C₁-C₆-Alkandiolen, insbesondere mit 2-Amino-2-methylpropan-1-ol, 2-Amino-2-methylpropan-1,3-diol, 2-Aminopropan-1-ol, 3-Aminopropan-1-ol, 1-Aminopropan-2-ol (MIPA) und 2-Amino-2-(hydroxymethyl)propan-1,3-diol (TRIS), wobei die Salze mit 2-Amino-2-methylpropan-1-ol und 2-Amino-2-methylpropan-1,3-diol besonders bevorzugt sind.

Außerordentlich bevorzugt ist das Phenylen-1,4-bis-(2-benzimidazyl)-3,3'-5,5'-tetrasulfonsäure-bis-natriumsalz mit der INCI-Bezeichnung "Disodium Phenyl Dibenzimidazole Tetrasulfonate" (CAS-Nr.: 180898-37-7), das beispielsweise unter der Handelsbezeichnung Neo Heliopan AP von Symrise erhältlich ist. Weitere bevorzugte wasserlösliche organische UV-Filtersubstanzen sind die 2-Phenylbenzimidazol-5-sulfonsäure (INCI-Bezeichnung "Phenylbenzimidazole sulfonic acid" (CAS.-Nr. 27503-81-7), beispielsweise unter den Handelsnamen Neo Heliopan Hydro von Symrise oder Eusolex 232 von Merck KGaA erhältlich; UV-B-Filter) und ihre Salze, insbesondere die Alkali-, Erdalkali-, Ammonium-, Alkylammonium-, Alkanolamin- und Glucammoniumsalze, bevorzugt die entsprechenden Natrium-, Kalium-, Trialkylammonium- oder Triethanolamin-Salze, weiterhin die Salze dieser Säure mit Amino-C₁-C₆-Alkanolen und Amino-C₁-C₆-Alkandiolen, insbesondere mit 2-Amino-2-methylpropan-1-ol, 2-Amino-2-methylpropan-1,3-diol, 2-Aminopropan-1-ol, 3-Aminopropan-1-ol und 1-Aminopropan-2-ol (MIPA), wobei die Salze mit 2-Amino-2-methylpropan-1-ol und 2-Amino-2-methylpropan-1,3-diol besonders bevorzugt sind, insbesondere aber die Salze der 2-Phenylbenzimidazol-5-sulfonsäure mit Natriumionen und mit 2-Amino-2-methylpropan-1-ol oder 2-Amino-2-methylpropan-1,3-diol.

In einer besonders bevorzugten Ausführungsform der Erfindung ist der mindestens eine wasserlösliche organische UV-Filter, der ein Derivat der Benzimidazolsulfonsäure darstellt, ausgewählt aus Phenylen-1,4-bis-(2-benzimidazyl)-3,3'-5,5'-tetrasulfonsäure, 2-Phenylbenzimidazol-5-sulfonsäure und den Salzen dieser Säuren, bevorzugt den entsprechenden Natrium-, Kalium- oder Triethanolamin-Salzen, insbesondere aus Phenylbenzimidazolsulfonsäure und dem Phenylen-1,4-bis-(2-benzimidazyl)-3,3'-5,5'-tetrasulfonsäure-bis-natriumsalz.

Bevorzugte erfindungsgemäße Öl-in-Wasser-Emulsionen sind **dadurch gekennzeichnet, dass** das/die Derivat/e der Benzimidazolsulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, enthalten ist/sind, jeweils bezogen auf das Gesamtgewicht der erfindungsgemäßen Zusammensetzung.

Die Gewichtsangabe bezieht sich dabei, unabhängig von der Form, in der das Benzimidazolsulfonsäure-Derivat vorliegt (Salz, freie Säure), auf das Gewicht des Benzimidazolsulfonsäure-Derivats in der Säureform.

Weitere bevorzugte erfindungsgemäße Öl-in-Wasser-Emulsionen sind **dadurch gekennzeichnet, dass** 2-Phenylbenzimidazol-5-sulfonsäure oder ein Salz hiervon in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, enthalten ist/sind, jeweils bezogen auf das Gesamtgewicht der erfindungsgemäßen Zusammensetzung.

Weitere bevorzugte erfindungsgemäße Öl-in-Wasser-Emulsionen sind **dadurch gekennzeichnet, dass** Phenylen-1,4-bis-(2-benzimidazyl)-3,3'-5,5'-tetrasulfonsäure oder ein Salz hiervon in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, enthalten ist/sind, jeweils bezogen auf das Gesamtgewicht der erfindungsgemäßen Zusammensetzung, wobei die Gesamtmenge aller Benzimidazolsulfonsäure-Derivate 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, beträgt.

Weitere bevorzugte erfindungsgemäße Öl-in-Wasser-Emulsionen sind **dadurch gekennzeichnet, dass** Phenylen-1,4-bis-(2-benzimidazyl)-3,3'-5,5'-tetrasulfonsäure und 2-Phenylbenzimidazol-5-sulfonsäure oder Salze hiervon in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, enthalten ist/ sind, jeweils bezogen auf das Gesamtgewicht der erfindungsgemäßen Zusammensetzung, wobei die Gesamtmenge aller Benzimidazolsulfonsäure-Derivate 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, beträgt.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest und weiterhin Polysilicone-15 enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest in einer Gesamtmenge von 0,5 Gew.-% bis 8 Gew.-%, besonders bevorzugt 1 bis 6 Gew.-%, insbesondere 2,5 - 4,5 Gew.-% und außerordentlich bevorzugt 3 - 4 Gew.-% und weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest, weiterhin Polysilicone-15 und Natrium-2-Phenylbenzimidazol-5-sulfonsäure enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest in einer Gesamtmenge von 0,5 Gew.-% bis 8 Gew.-%, besonders bevorzugt 1 bis 6 Gew.-%, insbesondere 2,5 - 4,5 Gew.-% und außerordentlich bevorzugt 3 - 4 Gew.-%, weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, und Natrium-2-Phenylbenzimidazol-5-sulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest, weiterhin Polysilicone-15 und Natrium-2-Phenylbenzimidazol-5-sulfonsäure enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest in einer Gesamtmenge von 0,5 Gew.-% bis 8 Gew.-%, besonders bevorzugt 1 bis 6 Gew.-%, insbesondere 2,5 - 4,5 Gew.-% und außerordentlich bevorzugt 3 - 4 Gew.-%, weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, und Natrium-2-Phenylbenzimidazol-5-sulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest, weiterhin Polysilicone-15, das Natriumsalz und das 2-Amino-2-methylpropan-l-ol-Salz der 2-Phenylbenzimidazol-5-sulfonsäure enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest in einer Gesamtmenge von 0,5 Gew.-% bis 8 Gew.-%, besonders bevorzugt 1 bis 6 Gew.-%, insbesondere 2,5 - 4,5 Gew.-% und außerordentlich bevorzugt 3 - 4 Gew.-%, weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, das Natriumsalz und das 2-Amino-2-methylpropan-1-ol-Salz der 2-Phenylbenzimidazol-5-sulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen.

Gemäß den Herstellerangaben aus WO 2007/135196 A2 lassen sich die vorgenannten Derivate der Benzimidazolsulfonsäure gegenüber einer Kristallisation besonders gut durch Neutralisation mit basischen Aminosäuren, insbesondere mit Ornithin, Lysin, Arginin und/oder Histidin stabilisieren, wobei Arginin besonders bevorzugt ist. Dies konnte für den Gegenstand der vorliegenden Anmeldung nicht bestätigt werden.

In einer bevorzugten Ausführungsform enthalten die erfindungsgemäßen Öl-in-Wasser-Emulsionen daher basische Aminosäuren, wie Lysin, Arginin und Histidin, in einer Gesamtmenge von 0 bis maximal 0,1 Gew.-%, bezogen auf die gesamte Zusammensetzung.

Eine Stabilisierung des Lichtschutzfaktors (SPF) im vorstehend angegebenen pH-Bereich in Kombination mit dem mindestens einen linearen, gesättigten primären Alkohol mit 14 - 30 Kohlenstoffatomen und dem mindestens einen Alkylglycosid mit einem linearen oder verzweigten Alkyl- oder Alkenylrest mit 14 - 30 Kohlenstoffatomen und einem Mono-, Di- oder Triglycosidrest konnte insbesondere beobachtet werden, wenn neben dem öllöslichen organischen UV-Filter Polysilicone-15 weiterhin mindestens ein Alkyl- und/oder Alkoxy-substituiertes Dibenzoylmethanderivat als öllöslicher organischer UV-Filter enthalten ist.

In einer bevorzugten Ausführungsform der Erfindung sind die erfindungsgemäßen kosmetischen Öl-in-Wasser-Emulsionen **dadurch gekennzeichnet, dass** der mindestens eine öllösliche UV-Filter eine Kombination aus mindestens einem Alkyl- und/oder Alkoxy-substituierten Dibenzoylmethanderivat und Polysilicone-15 umfasst.

Alkyl- und/oder Alkoxy-substituiertes Dibenzoylmethanderivat

Erfindungsgemäß bevorzugte Alkyl- und/oder Alkoxy-substituierte Dibenzoylmethanderivate sind **dadurch gekennzeichnet, dass** einer der Phenylreste mit mindestens einer Alkylgruppe, ausgewählt aus Methyl, Ethyl, n-Propyl, Isopropyl, n-Butyl, sec-Butyl, tert.-Butyl, n-Pentyl, Isopentyl, Neopentyl, 2-Ethylhexyl, und der andere Phenylrest mit mindestens einer Alkoxy-Gruppe, ausgewählt aus Methoxy, Ethoxy, n-Propoxy, Isopropoxy, n-Butoxy, sec-Butoxy, tert.Butoxy, n-Pentoxy, Isopentoxy, Neopentoxy, 2-Ethylhexoxy, substituiert ist. Besonders bevorzugte Substituenten sind Isopropyl, tert.-Butyl und Methoxy. Erfindungsgemäß bevorzugte Alkyl- und/oder Alkoxy-substituierte Dibenzoylmethanderivate sind ausgewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan. Besonders bevorzugt ist 4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet), ein öllöslicher organischer Lichtschutzfilter, der z. B. als Parsol^{®} 1789 von DSM oder als Eusolex^{®} 9020 von Merck KGaA erhältlich ist.

Bevorzugte erfindungsgemäße Öl-in-Wasser-Emulsionen sind **dadurch gekennzeichnet, dass** das mindestens eine Alkyl- und/oder Alkoxy-substituierte Dibenzoylmethanderivat in einer Gesamtmenge von 0,5 Gew.-% bis 20 Gew.-%, bevorzugt 1 bis 15 Gew.-%, besonders bevorzugt 2 bis 10 Gew.-% und außerordentlich bevorzugt 3 - 5 Gew.-%, enthalten ist, jeweils bezogen auf das Gesamtgewicht der erfindungsgemäßen Zusammensetzung.

Weitere bevorzugte erfindungsgemäße Öl-in-Wasser-Emulsionen sind **dadurch gekennzeichnet, dass** das mindestens eine Alkyl- und/oder Alkoxy-substituiertes Dibenzoylmethanderivat ausgewählt ist aus 4-(tert.-Butyl)-4'-methoxydibenzoylmethan.

Weitere bevorzugte erfindungsgemäße Öl-in-Wasser-Emulsionen sind **dadurch gekennzeichnet, dass** 4-(tert.-Butyl)-4'-methoxydibenzoylmethan in einer Gesamtmenge von 0,5 Gew.-% bis 20 Gew.-%, bevorzugt 1 bis 15 Gew.-%, besonders bevorzugt 2 bis 10 Gew.-% und außerordentlich bevorzugt 3 - 5 Gew.-%, enthalten ist, jeweils bezogen auf das Gesamtgewicht der erfindungsgemäßen Zusammensetzung.

Weitere bevorzugte erfindungsgemäße Öl-in-Wasser-Emulsionen sind **dadurch gekennzeichnet, dass** a) 4-(tert.-Butyl)-4'-methoxydibenzoylmethan, b) Polysilicone-15 und c) 2-Phenylbenzimidazol-5-sulfonsäure(salz) enthalten sind.

Weitere bevorzugte erfindungsgemäße Öl-in-Wasser-Emulsionen sind **dadurch gekennzeichnet, dass** a) 4-tert-Butyl-4'-Methoxydibenzoylmethan, b) Polysilicone-15 und c) Dinatriumphenylbenzimidazoltetrasulfonat enthalten sind.

Weitere bevorzugte erfindungsgemäße Öl-in-Wasser-Emulsionen sind **dadurch gekennzeichnet, dass** die UV-Filter a), b) und c) in einem Gewichtsverhältnis zueinander von a) : b) : c) wie (0,8 - 1,5) : (0,8 - 1,5) : (0,8 - 1,5) enthalten sind.

Weitere bevorzugte erfindungsgemäße Öl-in-Wasser-Emulsionen sind **dadurch gekennzeichnet, dass** die UV-Filter a), b) und c) in einem Gewichtsverhältnis zueinander von a) : b) : c) wie (0,9 - 1,2) : (0,9 - 1,2) : (0,9 - 1,2), bevorzugt (1 - 1, 1) : (1 - 1, 1) : (1 - 1, 1), enthalten sind.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest, weiterhin Polysilicone-15, 4-tert-Butyl-4'-Methoxydibenzoylmethan und das Natriumsalz der 2-Phenylbenzimidazol-5-sulfonsäure enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest in einer Gesamtmenge von 0,5 Gew.-% bis 8 Gew.-%, besonders bevorzugt 1 bis 6 Gew.-%, insbesondere 2,5 - 4,5 Gew.-% und außerordentlich bevorzugt 3 - 4 Gew.-%, weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, 4-tert-Butyl-4'-Methoxydibenzoylmethan in einer Gesamtmenge von 0,5 Gew.-% bis 20 Gew.-%, bevorzugt 1 bis 15 Gew.-%, besonders bevorzugt 2 bis 10 Gew.-% und außerordentlich bevorzugt 3 - 5 Gew.-% und das Natriumsalz der 2-Phenylbenzimidazol-5-sulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest, weiterhin Polysilicone-15, 4-tert-Butyl-4'-Methoxydibenzoylmethan, das Natriumsalz und das 2-Amino-2-methylpropan-1-ol-Salz der 2-Phenylbenzimidazol-5-sulfonsäure enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest in einer Gesamtmenge von 0,5 Gew.-% bis 8 Gew.-%, besonders bevorzugt 1 bis 6 Gew.-%, insbesondere 2,5 - 4,5 Gew.-% und außerordentlich bevorzugt 3 - 4 Gew.-%, weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, 4-tert-Butyl-4'-Methoxydibenzoylmethan in einer Gesamtmenge von 0,5 Gew.-% bis 20 Gew.-%, bevorzugt 1 bis 15 Gew.-%, besonders bevorzugt 2 bis 10 Gew.-% und außerordentlich bevorzugt 3 - 5 Gew.-%, das Natriumsalz und das 2-Amino-2-methylpropan-1-ol-Salz der 2-Phenylbenzimidazol-5-sulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen.

### s-Triazinderivate

Überraschend wurde festgestellt, dass mit öllöslichen organischen UV-Filtersubstanzen auf Basis von s-Triazinderivaten, die das nachfolgende Strukturmotiv 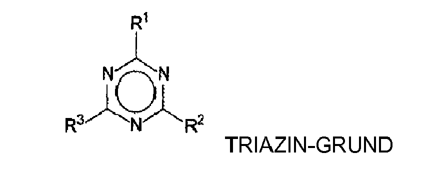 aufweisen, mit dem obligatorischen O/W-Emulgatorsystem, umfassend ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest, im obligatorischen pH-Bereich teilweise geringere SPF-Werte und geringere Lagerstabilitäten erzielt werden konnten als beispielsweise mit Polysilicone-15. In einer bevorzugten Ausführungsform sind die erfindungsgemäßen Öl-in-Wasser-Emulsionen daher frei von s-Triazinderivaten, die das oben stehende Strukturmotiv TRIAZIN-GRUND aufweisen.

s-Triazinderivate, die das oben stehende Strukturmotiv TRIAZIN-GRUND aufweisen, sind im Stand der Technik bekannt, beispielsweise aus EP 775698, EP 878469 und EP 1027881.

Hinsichtlich der C₃-Achse des Triazin-Grundkörpers dieser Verbindungen sind sowohl symmetrische Substitution als auch unsymmetrische Substitution denkbar.

In diesem Sinne symmetrisch substituierte s-Triazine weisen drei gleiche Substituenten R¹, R² und R³ auf, während unsymmetrisch substituierte s-Triazinderivate demzufolge unterschiedliche Substituenten aufweisen, wodurch die C₃-Symmetrie zerstört wird. Im Sinne der vorliegenden Erfindung wird als "unsymmetrisch" stets unsymmetrisch hinsichtlich der C₃-Achse des Triazin-grundkörpers verstanden, es sei denn, etwas anderes wäre ausdrücklich erwähnt.

Hinsichtlich der C₃-Achse des Triazin-Grundkörpers symmetrische Triazinderivate sind beispielsweise solche der allgemeinen Formel TRIAZIN-GRUND mit R¹ = R² = R³ = -NH-Phe-COOR, mit Phe = Phenyl-Rest, bei dem die Substituenten -NH und -COOR in para-Position zueinander stehen. Entsprechende symmetrische Triazinderivate sind 4,4',4"-(1,3,5-Triazin-2,4,6-triyltriimino)-tris-benzoesäure-tris(alkylester), insbesondere 4,4',4"-(1,3,5-Triazin-2,4,6-triyltriimino)-tris-benzoesäure-tris(2-ethylhexylester) [INCI: Ethylhexyl Triazone, vormals Octyl Triazone], das als Einzelsubstanz von BASF unter dem Handelsnamen UVINUL® T 150 vertrieben wird, aber auch in diversen kommerziellen UV-Filtermischungen erhältlich ist.

Unsymmetrische Triazinderivate sind beispielsweise solche, die in EP 775698 offenbart sind: 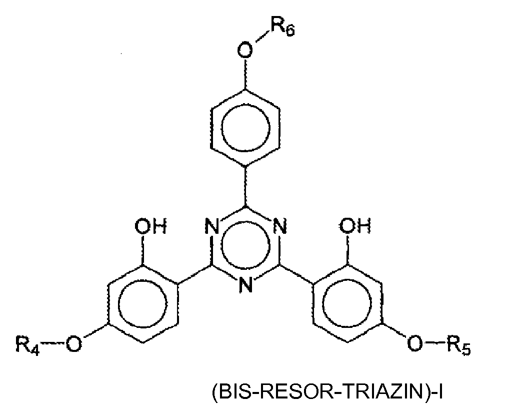 und/oder

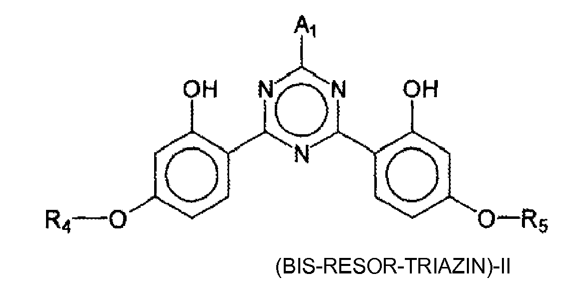

R₄ und R₅ können aus der Gruppe der verzweigten und unverzweigten Alkylgruppen von 1 bis 18 Kohlenstoffatomen gewählt. Auch können die Alkylgruppen wiederum mit Silyloxygruppen substituiert sein.

A₁ stellt beispielsweise einen substituierten homo- oder heterocyclischen aromatischen Fünfring oder Sechsring dar.

In der allgemeinen Formel (BIS-RESOR-TRIAZIN)-I kann R₆ ein Wasserstoffatom oder eine verzweigte oder unverzweigte Alkylgruppe mit 1 bis 10 Kohlenstoffatomen darstellen. Eine beispielhafte Verbindung der allgemeinen Formel (BIS-RESOR-TRIAZIN)-I, in der R₄ und R₅ jeweils eine 2-Ethylhexyl-Gruppe und R₆ eine Methylgruppe darstellen, ist 2,4-Bis-{[4-(2-ethylhexyloxy)-2-hydroxy]-phenyl}-6-(4-methoxyphenyl)-1,3,5-triazin (INCI: Bis-Ethylhexyloxyphenol Methoxyphenyl Triazine), unter dem Handelsnamen Tinosorb^{®} S von CIBA erhältlich.

Eine weitere beispielhafte unsymmetrisch substituierte s-Triazin-Verbindung ist eine Verbindung mit der INCI-Bezeichnung Diethylhexyl Butamido Triazone, mit der allgemeinen Formel TRIAZIN-GRUND mit R¹ = R² -NH-Phe-COOR, mit Phe = Phenyl-Rest, bei dem die Substituenten -NH und -COOR in para-Position zueinander stehen, R = 2-Ethylhexyl und R³ = -NH-Phe-CONH-tert-Butyl, mit Phe = Phenyl-Rest, bei dem die Substituenten -NH und -CONH-tert.-Butyl in para-Position zueinander stehen. Diese UV-Filtersubstanz, ein UV-A-Filter, ist unter der Handelsbezeichnung UVASORB HEB bei Sigma 3V erhältlich ist.

Weitere UV-Filtersubstanzen auf Basis von s-Triazin-Verbindungen sind:
- 2,4,6-Tris([1,1'-Biphenyl]-4-yl)-1,3,5-Triazine, INCI: Tris-Biphenyl Triazine, erhältlich unter dem Handelsnamen Tinosorb A2B von CIBA,
- 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma, INCI: Ethylhexyl Bis-Isopentylbenzoxazolylphenyl Melamine),
- 2,4-Bis-{[4-(3-sulfonato)-2-hydroxy-propyloxy)-2-hydroxy]-phenyl}-6-(4-methoxyphenyl)-1,3,5-triazin-Natriumsalz,
- 2,4-Bis-{[4-(3-(2-propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(ethylcarboxyl)-phenylamino]-1, 3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-([4-(2-methylpropenyloxy)-2-hydroxy]-phenyl}-6-(4-methoxyphenyl)-1,3 ,5-triazin und
- 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin,
- 2-[4,6-Bis(2,4-dimethylphenyl)-1,3,5-triazin-2-yl]-5-(ethylhexyloxy)phenol.

Weitere bevorzugte erfindungsgemäße Zusammensetzungen sind gekennzeichnet durch einen Gehalt an mindestens einer Booster-Substanz für den Lichtschutzfaktor.

Unter einer Booster-Substanz für den Lichtschutzfaktor wird im Sinne der vorliegenden Anmeldung eine Substanz verstanden, die, für sich genommen, keinen Lichtschutzfaktor (SPF) gegenüber UV-A- und UV-B-Strahlung aufweist, die aber in der Lage ist, den Lichtschutzfaktor (SPF) einer kosmetischen Zusammensetzung, die mindestens einen organischen oder anorganischen UV-Filter enthält, zu steigern, ohne dass hierzu eine Konzentrationserhöhung des UV-Filters nötig ist.

Im Rahmen der dieser Anmeldung zu Grunde liegenden Arbeiten wurden folgende Substanzen bzw. Substanzklassen als geeignete Booster-Substanz identifiziert:
i) Dicarbonsäureester von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat,
ii) 2-Propylheptyloctanoat,
iii) Natriumacrylat-Homopolymere mit einem mittleren Molgewicht von 2500000 bis 3500000 Dalton, deren 1 Gew.-%-ige wässrige Lösung einen pH-Wert von 5,5 - 7,5 aufweist,
iv) Siliciumdioxid-Partikel, die nicht organisch oder anorganisch modifiziert sind und keine organische oder anorganische Beschichtung aufweisen und zu mehr als 80 Gew.-% sphärische Partikel mit einem zahlenmittleren Partikeldurchmesser im Bereich von 2 - 15 µm enthalten.

Überraschend wurde festgestellt, dass mit der mindestens einen SPF-Booster-Substanz im Zusammenwirken mit einem pH-Wert im lediglich schwach sauren bis neutralen oder sogar leicht basischen Bereich eine deutlich verbesserte Langzeitstabilität des anfänglichen SPF erzielt wird.

### Booster

Für die erfindungsgemäßen Öl-in-Wasser-Emulsionen, die durch ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest, bei einem pH-Wert bei 20°C von 6,0 - 8, bevorzugt 6,5 - 7,5, besonders bevorzugt 6,8 bis 7,3, stabilisiert sind und die mindestens einen öllöslichen oder wasserlöslichen organischen UV-Filter enthalten, haben sich überraschend folgende Substanzen bzw. Substanzklassen als SPF-steigernde Substanzen ("Booster") erwiesen:
i) Dicarbonsäureester von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.
   Bevorzugte erfindungsgemäße Öl-in-Wasser-Emulsionen sind **dadurch gekennzeichnet, dass** sie mindestens einen Dicarbonsäureester von linearen oder verzweigten C₂-C₁₀-Alkanolen in einer Gesamtmenge von 0,1 - 20 Gew.-%, bevorzugt 1 - 10 Gew.-% und besonders bevorzugt 3 - 7 Gew.-%, enthalten, wobei Gesamtmengen von 3,5, 4, 4,5, 5, 5,5, 6 und 6,5 Gew.-% außerordentlich bevorzugt sind. Alle Gew.-%-Angaben beziehen sich auf das Gesamtgewicht der erfindungsgemäßen Öl-in-Wasser-Emulsion.
ii) 2-Propyiheptyioctanoat.
   Bevorzugte erfindungsgemäße Öl-in-Wasser-Emulsionen sind **dadurch gekennzeichnet, dass** sie 2-Propylheptyloctanoat in einer Gesamtmenge von 0,01 - 10 Gew.-%, bevorzugt 0,1 - 5 Gew.-% und besonders bevorzugt 0,5 - 3 Gew.-%, enthalten, wobei Gesamtmengen von 1, 1,5, 2 und 2,5 Gew.-% außerordentlich bevorzugt sind. Alle Gew.-%-Angaben beziehen sich auf das Gesamtgewicht der erfindungsgemäßen Öl-in-Wasser-Emulsion.
iii) Natriumacrylat-Homopolymere mit einem mittleren Molgewicht von 2500000 bis 3500000 Dalton, deren 1 Gew.-%-ige wässrige Lösung einen pH-Wert von 5,5 - 7,5 aufweist.
   Überraschend wurde festgestellt, dass der SPF erfindungsgemäßer Öl-in-Wasser-Emulsionen durch mindestens ein Natriumacrylat-Homopolymer mit einem mittleren Molgewicht von 2500000 bis 3500000 Dalton, dessen 1 Gew.-%-ige wässrige Lösung einen pH-Wert von 5,5 - 7,5 aufweist, gesteigert werden kann. Derartige Natriumacrylat-Homopolymere sind kommerziell erhältlich und werden von den Herstellern als emulsionsstabilisierendes, verdickendes Polymer angeboten. Ohne an diese Theorie gebunden sein zu wollen, wird vermutet, dass der relativ hohe pH-Wert dieses Booster-Polymers zur Stabilisierung und Steigerung des Lichtschutzfaktors beiträgt. Darüber hinaus wurde beobachtet, dass Booster dieser Substanzklasse den erfindungsgemäßen Öl-in-Wasser-Emulsionen ein außergewöhnlich gutes, nicht-klebriges und weiches Hautgefühl verleihen. Ein besonders bevorzugtes Natriumacrylat-Homopolymer mit einem mittleren Molgewicht von 2500000 bis 3500000 Dalton, dessen 1 Gew.-%-ige wässrige Lösung einen pH-Wert von 5,5 - 7,5 aufweist, ist unter dem Handelsnamen Cosmedia SP von der Firma Cognis erhältlich. Bevorzugte erfindungsgemäße Öl-in-Wasser-Emulsionen sind **dadurch gekennzeichnet, dass** sie mindestens ein Natriumacrylat-Homopolymer mit einem mittleren Molgewicht von 2500000 bis 3500000 Dalton, dessen 1 Gew.-%-ige wässrige Lösung einen pH-Wert von 5,5 - 7,5 aufweist, in einer Gesamtmenge von 0,01 - 5 Gew.-%, bevorzugt 0,1 - 3 Gew.-% und besonders bevorzugt 0,4 - 1,5 Gew.-%, enthalten, wobei Gesamtmengen von 0,5, 0,8 und 1,2 Gew.-% außerordentlich bevorzugt sind. Alle Gew.-%-Angaben beziehen sich auf das Gesamtgewicht der erfindungsgemäßen Öl-in-Wasser-Emulsion.
iv) Siliciumdioxid-Partikel, die nicht organisch oder anorganisch modifiziert sind und keine organische oder anorganische Beschichtung aufweisen und zu mehr als 80 Gew.-% sphärische Partikel mit einem zahlenmittleren Partikeldurchmesser im Bereich von 2 - 15 µm enthalten. Bevorzugte Siliciumdioxid-Partikel des vorgenannten Typs sind weiterhin gekennzeichnet durch mindestens eine weitere der folgenden Eigenschaften:
   zahlenmittlerer Partikeldurchmesser im Bereich von 5 - 10 µm, Ölabsorptionskapazität von 0,7 - 1,5 cm³ flüssiges Paraffin pro Gramm trockenes Siliciumdioxid, aufweisen, gemessen nach DIN 53601, erhältlich durch Kieselsäure-Fällung.
   Bevorzugte Siliciumdioxid-Partikel des vorgenannten Typs sind nicht organisch oder anorganisch modifiziert, weisen keine organische oder anorganische Beschichtung auf, enthalten zu mehr als 80 Gew.-% sphärische Partikel mit einem zahlenmittleren Partikeldurchmesser im
   Bereich von 5- 10 µm und wurden durch Kieselsäure-Fällung erhalten.
   Weitere bevorzugte Siliciumdioxid-Partikel des vorgenannten Typs sind nicht organisch oder anorganisch modifiziert, weisen keine organische oder anorganische Beschichtung auf, enthalten zu mehr als 80 Gew.-% sphärische Partikel mit einem zahlenmittleren Partikeldurchmesser im Bereich von 5 - 10 µm, wurden durch Kieselsäure-Fällung erhalten und weisen eine Ölabsorptionskapazität von 0,7 - 1,5 cm³ flüssiges Paraffin pro Gramm trockenes Siliciumdioxid, auf, gemessen nach DIN 53601.
   Bevorzugte Siliciumdioxid-Partikel des vorgenannten Typs sind kommerziell erhältlich, beispielsweise als Handelsprodukt SB-705 der Firma Miyoshi Kasei, einem sphärischen Kieselgel mit der INCI-Bezeichnung Silica, das einen zahlenmittleren Teilchendurchmesser von 5 - 6 µm und eine spezifische Oberfläche von etwa 600 m²/g aufweist. Weitere bevorzugte Siliciumdioxid-Partikel sind als Handelsprodukt Aerosil von Evonik Degussa erhältlich: Aerosil 130, Aerosil 200, Aerosil 255, Aerosil 300 oder Aerosil 380. Weitere bevorzugte Siliciumdioxid-Partikel sind ebenfalls als Handelsprodukte erhältlich: CAB-O-SIL Fumed Silica (Cabot), CAB-O-SIL EH-5 (Cabot), CAB-O-SIL HS-5 (Cabot), CAB-O-SIL LM-130 (Cabot), CAB-O-SIL MS-55 (Cabot), CAB-O-SIL M-5 (Cabot), Cosmedia Silc (Cognis), Neosil PC 50 S (Ineos Silicas), Sorbosil BFG 54 (Ineos Silicas), Sorbosil AC33 (Ineos Silicas), Sorbosil AC 35 (Ineos Silicas), Sorbosil AC 37 (Ineos Silicas), Sorbosil AC 39 (Ineos Silicas), Wacker HDK H 30 (Wacker-Chemie), Wacker HDK N 20 (Wacker-Chemie), Wacker HDK S 13 (Wacker-Chemie), Wacker HDK T 30 (Wacker-Chemie), Wacker HDK V 15 (Wacker-Chemie). Besonders bevorzugte Siliciumdioxid-Partikel der vorgenannten Typen sind unter dem Handelsnamen Cosmedia Silc von der Firma Cognis erhältlich.
   Bevorzugte erfindungsgemäße Zusammensetzungen sind **dadurch gekennzeichnet, dass** sie die Siliciumdioxid-Partikel in einer Gesamtmenge von 0,01 - 10 Gew.-%, bevorzugt 0,1 - 5 Gew.-%, besonders bevorzugt 1 - 4 Gew.-%, außerordentlich bevorzugt 1,5 - 3 Gew.-% und weiter bevorzugt 2 - 2,5 Gew.-%, jeweils bezogen auf die gesamte Zusammensetzung, enthalten.

Weitere bevorzugte erfindungsgemäße Zusammensetzungen sind **dadurch gekennzeichnet, dass** sie mindestens zwei der vorgenannten Booster-Substanzen enthalten. Besonders bevorzugt sind dabei Kombinationen von mindestens einer Booster-Substanz iii) und mindestens einer Booster-Substanz iv).

Weitere bevorzugte erfindungsgemäße Zusammensetzungen sind **dadurch gekennzeichnet, dass** sie mindestens drei der vorgenannten Booster-Substanzen enthalten. Besonders bevorzugt sind dabei Kombinationen von 2-Propylheptyloctanoat mit mindestens einer Booster-Substanz iii) und mindestens einer Booster-Substanz iv).

Weitere bevorzugte erfindungsgemäße Zusammensetzungen sind **dadurch gekennzeichnet, dass** sie mindestens vier der vorgenannten Booster-Substanzen enthalten. Besonders bevorzugt sind dabei Kombinationen, die 2-Propylheptyloctanoat und jeweils einen Vertreter der Booster-Substanzen i), iii) und iv) enthalten.

Um den Lichtschutzfaktor weiter zu erhöhen, können die erfindungsgemäßen Zusammensetzungen neben Polysilicone-15 und dem mindestens einen wasserlöslichen UV-Filter, umfassend ein Derivat der Benzimidazolsulfonsäure, bevorzugt mindestens eine weitere organische und/oder anorganische UV-Filtersubstanz enthalten.

Um den Lichtschutzfaktor weiter zu erhöhen, können die erfindungsgemäßen Zusammensetzungen bevorzugt mindestens eine weitere organische und/oder anorganische UV-Filtersubstanz enthalten, die besonders bevorzugt verschieden ist von s-Triazinderivaten, die das oben stehende Strukturmotiv TRIAZIN-GRUND aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest, weiterhin Polysilicone-15, 4-tert-Butyl-4'-Methoxydibenzoylmethan und das Natriumsalz der 2-Phenylbenzimidazol-5-sulfonsäure enthalten, einen pH-Wert bei 20°C von 6,0 - 8, bevorzugt 6,5 - 7,5, besonders bevorzugt 6,8 bis 7,3, aufweisen sowie frei sind von s-Triazinderivaten, die das oben stehende Strukturmotiv TRIAZIN-GRUND aufweisen.

Weitere bevorzugte öllösliche organische UV-Filtersubstanzen, die vorteilhaft in den erfindungsgemäßen Zusammensetzungen eingesetzt werden können, sind im Folgenden genannt.

### Benzotriazole

Eine weitere bevorzugte UV-Filtersubstanz ist 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol, MBBT) mit folgender Strukturformel 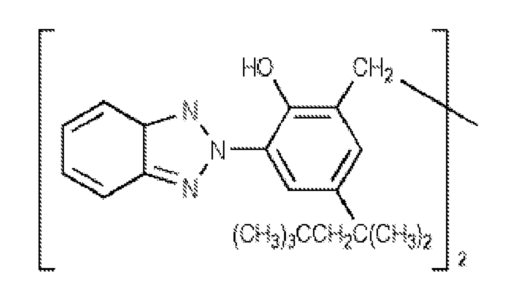 , unter der Handelsbezeichnung Tinosorb^{®} M von CIBA erhältlich. Hierbei handelt es sich um einen so genannten Breitbandfilter, der sowohl UV-A- als auch UV-B-Strahlung absorbiert.

Eine weitere bevorzugte optionale Breitband-UV-Filtersubstanz ist 2-(2H-Benzotriazol-2-yl)-4-methyl-6-[2-methyl-3-[1,3,3,3-tetramethyl-1-((trimethylsilyl)oxy]disiloxanyl]propyl]-phenol (CAS-Nr.: 155633-54-8) mit der INCI-Bezeichnung Drometrizole Trisiloxane.

Weitere bevorzugte optionale Benzotriazol-Filter sind 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4) und 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane.

Weitere bevorzugte UV-Filtersubstanzen, die neben denen der Komponenten (a), (b) und (c) in den erfindungsgemäßen Zusammensetzungen eingesetzt werden, sind im Folgenden genannt:
- Derivate des Camphers, insbesondere 3-Benzylidencampher-Derivate, die keine ionisierbaren funktionellen Gruppen im Molekül aufweisen können, bevorzugt 3-(4'-Methylbenzyliden)-D,L-campher [INCI: 4-Methylbenzylidene Camphor];
- Derivate des Camphers, die ionisierbare funktionelle Gruppen im Molekül aufweisen können, bevorzugt Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze; das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolamin-Salz), das auch als Benzol-1,4-di(2-oxo-3-bornylidenmethyl-10-sulfonsäure) bezeichnet wird, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalylidene Dicamphor Sulfonic Acid (CAS.-Nr.: 92761-26-7, als Mexoryl SX von Chimex erhältlich);
- 4-Aminobenzoësäure-Derivate, bevorzugt 4-(Dimethylamino)-benzoësäure(2-ethylhexyl) ester, 4-(Dimethylamino)benzoësäureamylester;
- 2-Aminobenzoësäure-Derivate;
- Ester der Zimtsäure, bevorzugt 4-Methoxyzimtsäure(2-ethylhexyl)ester, 4-Methoxyzimtsäurepropylester, 4-Methoxyzimtsäureisopentylester; und 2-Cyano-3,3-phenylzimtsäure-2-ethylhexylester (= Ethylhexyl-2-cyano-3,3-diphenylacrylat (Octocrylene)),
- Ester der Salicylsäure, bevorzugt Salicylsäure(2-ethylhexyl)ester (2-Ethylhexylsalicylat (= Octylsalicylat)), Salicylsäure(4-isopropylbenzyl)ester (4-Isopropylbenzylsalicylat), Salicylsäurehomomenthylester (Homomenthylsalicylat, Homosalate);
- Derivate des Benzophenons, die keine ionisierbaren funktionellen Gruppen im Molekül aufweisen, bevorzugt 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon, 2,2'-Dihydroxy-4-methoxybenzophenon; 2-(4'-Diethylamino-2'-hydroxybenzoyl)-benzoesäurehexylester (auch: Aminobenzophenon, unter der Bezeichnung Uvinul A Plus von BASF erhältlich),
- Derivate des Benzophenons, die ionisierbare funktionelle Gruppen im Molekül aufweisen, bevorzugt Sulfonsäurederivate von Benzophenonen, besonders bevorzugt 2-Hydroxy-4-methoxybenzophenon-5-sulfonsäure und ihre Salze,
- Ester der Benzalmalonsäure, bevorzugt 4-Methoxybenzalmalonsäuredi(2-ethylhexyl)ester,
- an Polymere gebundene UV-Filter, die von Polysilicone-15 verschieden sind;
- Derivate von Benzoxazol, bevorzugt 2,2'(Naphthalene-1,4-Diyl)bis(benzoxazole) (INCI: Dibenzoxazoyl Naphthalene) und 2,4-bis-[5-1(di-methylpropyl)benzoxazol-2-yl-(4-phenyl)-imino]-6-(2-ethylhexyl)-imino-1,3,5-triazin (Uvasorb^{®} K2A von 3V Sigma, INCI: Ethylhexyl Bis-Isopentylbenzoxazolylphenyl Melamine). Die Benzoxazol-Derivate liegen bevorzugt in gelöster Form in den erfindungsgemäßen kosmetischen Zusammensetzungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'-methoxyphenyl)propan-1,3-dion in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4`-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4`methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0, besonders bevorzugt bei 0,5 - 0,75, außerordentlich bevorzugt bei 0,6 - 0,9.

Die Liste der genannten optionalen UV-Filtersubstanzen, die im Sinne der vorliegenden Erfindung eingesetzt werden können, soll selbstverständlich nicht limitierend sein.

Die Gesamtmenge an der mindestens einen öllöslichen organischen UV-Filtersubstanz in den erfindungsgemäßen kosmetischen oder dermatologischen Zusammensetzungen beträgt bevorzugt 0,5 - 20 Gew.-%, besonders bevorzugt 1 - 15 Gew.-%, außerordentlich bevorzugt 2 - 10 Gew.-%, jeweils bezogen auf das Gesamtgewicht der erfindungsgemäßen Zusammensetzung.

Bei den erfindungsgemäß bevorzugten optionalen anorganischen UV-Filtersubstanzen handelt es sich bevorzugt um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und

Bariumsulfat. Besonders bevorzugt sind Titandioxid und Zinkoxid. Die Partikel sollten dabei einen mittleren Durchmesser von weniger als 100 nm, bevorzugt zwischen 5 und 50 nm und insbesondere zwischen 15 und 30 nm aufweisen, also so genannte Nanopigmente darstellen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane, bevorzugt Triethoxy Caprylylsilane, oder Simethicone in Frage. Weitere bevorzugte Coatingmittel sind Aluminiumoxide. Weitere bevorzugte Coatingmittel ist Siliciumdioxid, z. B. bei dem Handelsprodukt Eusolex T AVO von Merck KGaA. Ein weiteres bevorzugtes Coatingmittel ist Stearinsäure. Ein weiteres bevorzugtes Coatingmittel ist Polyhydroxystearinsäure. Ein weiteres bevorzugtes Coatingmittel ist Aluminiumstearat. Ein besonders bevorzugter anorganischer UV-Filter ist ein mit Triethoxy Caprylylsilane hydrophob beschichtetes Titandioxid, erhältlich unter der Bezeichnung Titan M 265 von Kemira. Erfindungsgemäß bevorzugt ist mindestens eine anorganische UV-Filtersubstanz in einer Gesamtmenge von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Um die haptischen Eigenschaften der erfindungsgemäßen Zusammensetzungen weiter zu verbessern, enthalten bevorzugte erfindungsgemäße Öl-in-Wasser-Emulsionen Arachylalkohol und/oder Behenylalkohol. Besonders bevorzugte erfindungsgemäße Öl-in-Wasser-Emulsionen sind **dadurch gekennzeichnet, dass** Arachylalkohol und/oder Behenylalkohol in einer Gesamtmenge von 2,5 - 4,5 Gew.-%, bevorzugt 3 - 4 Gew.-%, jeweils bezogen auf die gesamte Zusammensetzung, enthalten sind. Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest, weiterhin Polysilicone-15, 4-tert-Butyl-4'-methoxydibenzoylmethan, das Natriumsalz der 2-Phenylbenzimidazol-5-sulfonsäure, Arachylalkohol und/oder Behenylalkohol in einer Gesamtmenge von 2,5 - 4,5 Gew.-%, bevorzugt 3 - 4 Gew.-%, enthalten und einen pH-Wert bei 20°C von 6,0 - 8, bevorzugt 6,5 - 7,5, besonders bevorzugt 6,8 bis 7,3, aufweisen.

Weitere bevorzugte erfindungsgemäße Öl-in-Wasser-Emulsionen sind **dadurch gekennzeichnet, dass** sie ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest, weiterhin Polysilicone-15, 4-tert-Butyl-4'-Methoxydibenzoylmethan, das Natriumsalz der 2-Phenylbenzimidazol-5-sulfonsäure, Arachylalkohol und/oder Behenylalkohol in einer Gesamtmenge von 2,5 - 4,5 Gew.-%, bevorzugt 3 - 4 Gew.-%, enthalten und einen pH-Wert bei 20°C von 6,0 - 8, bevorzugt 6,5 - 7,5, besonders bevorzugt 6,8 bis 7,3, aufweisen sowie frei sind von s-Triazinderivaten, die das oben stehende Strukturmotiv TRIAZIN-GRUND aufweisen.

Um die haptischen Eigenschaften und/oder den Lichtschutzfaktor der erfindungsgemäßen Zusammensetzungen weiter zu verbessern, können die erfindungsgemäßen Zusammensetzungen bevorzugt mindestens einen teilchenförmigen Feststoff, ausgewählt aus färbenden, farbgebenden, mattierenden oder glanzgebenden Pigmenten. Bevorzugte Pigmente dieser Art können anorganisch oder organisch sein. Weitere bevorzugte Pigmente weisen einen zahlenmittleren Teilchendurchmesser von 0,1 - 200 µm, bevorzugt 0,5 - 100 µm, besonders bevorzugt 1 - 50 µm und außerordentlich bevorzugt 2 - 30 µm auf. Besonders bevorzugte anorganische Pigmente sind ausgewählt aus den Oxiden von Silicium, Titan, Eisen, Zink, Zirkonium, Magnesium, Cer und Bismut, aus Bismutoxychlorid, Bornitrid, Glimmer, Flussspat und wasserunlöslichen Perlglanzpigmenten, die mit mindestens einer anorganischen und/oder organischen Verbindung beschichtet sein können.

Erfindungsgemäß bevorzugte Perlglanzpigmente sind ausgewählt aus natürlichen Perlglanzpigmenten, wie z. B. "Fischsilber" (Guanin/Hypoxanthin-Mischkristalle aus Fischschuppen) und "Perlmutt" (gemahlene Muschelschalen), monokristallinen Perlglanzpigmenten, wie z. B. Bismuthoxychlorid (BiOCI), und Schicht-Substrat Pigmenten, z. B. Glimmer/Metalloxid. Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere bevorzugt ist z. B. das unter der CIN 77163 aufgelistete Glanzpigment.

Es kann andererseits erfindungsgemäß bevorzugt sein, gänzlich auf ein Substrat wie Glimmer zu verzichten. Besonders bevorzugt sind Perlglanzpigmente, die unter der Verwendung von SiO₂ hergestellt werden. Solche Pigmente, die auch zusätzlich goniochromatische Effekte haben können, sind z. B. unter dem Handelsnamen Sicopearl Fantastico bei der Firma BASF erhältlich.

Weitere bevorzugte Pigmente sind ausgewählt aus farbigen und farblosen Pigmenten. Einige der im Folgenden genannten Pigmente dienen auch als UV-Absorber. Besonders bevorzugte farbige Pigmente sind ausgewählt aus den Eisenoxiden mit den Color Index-Nummern CI 77491 (Eisenoxid rot), CI 77492 (Eisenoxidhydrat gelb) und CI 77499 (Eisenoxid schwarz), aus CI 77891 (Titandioxid) und Ruß. Ein besonders bevorzugtes Pigment ist das Handelsprodukt SUNPMMA-S und SUNSIL Tin 30 von Sunjin Chemicals Co. mit einem zahlenmittleren Teilchendurchmesser von 5 - 10 µm bzw. 2 - 7 µm.

Besonders bevorzugte optionale anorganische Pigmente sind beschichtet. Die Beschichtung kann mit Hilfe von anorganischen und/oder organischen Verbindungen erfolgen. Erfindungsgemäß besonders bevorzugt sind anorganische Pigmente, die eine anorganische Beschichtung aufweisen. Außerordentlich bevorzugte Pigmente dieser Art sind ausgewählt aus Siliciumdioxid-Partikeln, die mit Titandioxid und/oder Eisenoxiden beschichtet sind. Ein besonders bevorzugtes Pigment dieser Art ist das Handelsprodukt Ronasphere^{®} LDP der Firma Merck KGaA. Bei diesem Produkt handelt es sich um sphärische Siliciumdioxid-Partikel, die mit Titandioxid und Eisenoxid beschichtet sind. Ronasphere^{®} LDP weist einen zahlenmittleren Teilchendurchmesser von 4 - 7 µm auf.

Weiterhin bevorzugt sind anorganisch beschichtete Glimmerpigmente, die keinen Perlglanz aufweisen. Weitere bevorzugte optionale anorganisch beschichtete anorganische Pigmente sind Glimmerpigmente, die mit Titandioxid in verschiedenen Schichtdicken beschichtet sind, beispielsweise die Produkte der Timiron^{®}-Serie von Rona/Merck KGaA, insbesondere Pigmente der Produktreihen Timiron^{®} MP, Timiron^{®} Super, Timiron^{®} Starlight und Timiron^{®} Silk. Die genannten Produkte weisen mittlere Teilchendurchmesser von 5 - 60 µm bzw. 10 - 60 µm bzw. 10 - 125 µm bzw. 5 - 25 µm auf. Ebenfalls bevorzugt sind Glimmerpartikel mit einer Beschichtung aus Titandioxid und Eisenoxid, z. B. die Handelsprodukte Timiron^{®} MP-20, MP-24, MP-25, MP-28, MP-29, MP-60 und MP-65. Weiterhin erfindungsgemäß bevorzugt sind mit Titandioxid und/oder rotem und/oder schwarzem Eisenoxid beschichtete Glimmerpartikel, z. B. die Produkte der Colorona^{®}-Serie. Weitere bevorzugte Pigmente sind mit Kieselgel beschichtete Glimmerpigmente, z. B. das Handelsprodukt Micronasphere^{®} M. Weitere erfindungsgemäß bevorzugte Pigmente sind anorganisch beschichtete anorganische Pigmente, deren Beschichtung einen Anteil von 0,1 - 1 Gew.% Zinnoxid aufweist.

Ebenfalls erfindungsgemäß bevorzugt sind anorganische Pigmente, die mit organischen Substanzen beschichtet sind. Bevorzugte Beispiele hierfür sind mit Aluminiumstearat beschichtete Titandioxid-Pigmente (z. B. das Handelsprodukt MT 100 T von der Firma Tayca), mit Dimethylpolysiloxan (Dimethicone) beschichtetes Zinkoxid, mit Dimethicone beschichtetes Bornitrid (Tres BN^{®} UHP 1106 von Carborundum), mit einem Gemisch aus Dimethylpolysiloxan und Silicagel (Simethicone) und Aluminiumoxidhydrat (Alumina) beschichtetes Titandioxid (Eusolex^{®} T 2000 von Merck), mit Octylsilanol beschichtetes Titandioxid oder sphärische Polyalkylsesquisiloxan-Partikel (Aerosil^{®} R972 von Evonik). Weitere erfindungsgemäß besonders bevorzugte Zusammensetzungen sind **dadurch gekennzeichnet, dass** sie mindestens ein färbendes, farbgebendes, mattierendes oder glanzgebendes Pigment in einer Gesamtmenge von 0,1 Gew.-% bis 30 Gew.-%, bevorzugt 0,5 bis 15 Gew.-%, besonders bevorzugt 1,0 bis 10 Gew.-% und außerordentlich bevorzugt 2 - 5 Gew.-%, enthalten, jeweils bezogen auf das Gesamtgewicht der erfindungsgemäßen Zusammensetzung.

Weitere bevorzugte optionale teilchenförmige Feststoffe sind die durch Hydrolyse und Kondensationsreaktionen von Methyltrimethoxysilane erhältlichen Polymethylsilsesquioxane, die sphärisch sein können (insbesondere Aerosil^{®} R 972 und Aerosil^{®} 200 V von Evonik Degussa), und deren Teilchengrößenverteilung durch die Herstellung gesteuert werden kann.

Die optionalen teilchenförmigen Feststoffe können sowohl einzeln als auch im Gemisch eingesetzt werden.

Die Gesamtmenge der optionalen teilchenförmigen Feststoffe beträgt bevorzugt 0,1 - 30 Gew.-%, besonders bevorzugt 0,5 - 15 Gew.-%, außerordentlich bevorzugt 1,0 - 10 Gew.-%, oder auch 2 - 4 Gew.-%, jeweils bezogen auf das Gesamtgewicht der erfindungsgemäßen Zusammensetzung.

Die erfindungsgemäßen Zusammensetzungen können ferner kosmetische Hilfsstoffe und weitere Wirkstoffe enthalten, wie sie üblicherweise in solchen Zusammensetzungen verwendet werden, z. B. Konservierungsmittel, Konservierungshelfer, Bakterizide, Parfüms, Farbstoffe, Verdickungsmittel, anfeuchtende und/oder feuchthaltende Substanzen, Fette, Öle, Wachse, Alkohole, Polyole, Polymere, Elektrolyte, organische Lösemittel oder Siliconderivate.

Vorteilhafte Konservierungsmittel sind beispielsweise Formaldehydabspalter (wie z. B. DMDM Hydantoin), lodopropylbutylcarbamate, Parabene, Phenoxyethanol, Ethanol, Benzoesäure und andere. Bevorzugt umfasst das Konservierungssystem ferner auch Konservierungshelfer, wie beispielsweise Octoxyglycerin, 1,2-Pentandiol, 1,5-Pentandiol, 1,2-Hexandiol, 1,6-Hexandiol, 1,2-Heptandiol, 1,2-Octandiol, 1,8-Octandiol, 1,2-Nonandiol, 1,2-Decandiol, 1,10-Decandiol, 1,2-Undecandiol, 1,2-Dodecandiol, 1,2-Tridecandiol oder 1,2-Tetradecandiol.

Besonders bevorzugte Zusammensetzungen werden ferner erhalten, wenn als Zusatz- oder Wirkstoffe Antioxidantien eingesetzt werden. Erfindungsgemäß bevorzugt enthalten die Zusammensetzungen mindestens ein Antioxidans. Als günstige, aber dennoch fakultativ zu verwendende Antioxidantien können alle für kosmetische und/oder dermatologische Anwendungen geeigneten oder gebräuchlichen Antioxidantien verwendet werden, insbesondere Tocopherol, Tocopherylacetat oder Dimethylmethoxy Chromanol, erhältlich unter dem Handelsnamen Lipochroman-6 von Lipotec.

Weiter vorteilhaft werden der oder die Wirkstoffe gewählt aus der Gruppe, die Catechine und Gallensäureester von Catechinen und wässrige bzw. organische Extrakte aus Pflanzen oder Pflanzenteilen umfasst, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R, 3R)-3,3',4',5,7-Flavanpentaol) ist ein vorteilhafter Wirkstoff im Sinne der vorliegenden Erfindung.

In einer weiteren bevorzugten Ausführungsform enthalten die erfindungsgemäßen Zusammensetzungen mindestens ein Flavonoid oder mindestens einen Flavonoid-reichen Pflanzenextrakt. Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-a-L-mannopyranosyl)-β-D-glucopyranosid)), Monoxerutin (3,3',4',5-Tetrahydroxy-7-(2-hydroxyethoxy)-flavon-3-(6-O-(6-deoxy-a-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid). Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.

Erfindungsgemäß besonders bevorzugte Zusammensetzungen sind **dadurch gekennzeichnet, dass** sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-1) auf: 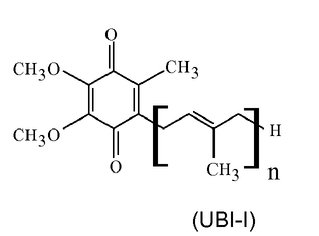 mit n = 6, 7, 8, 9 oder 10.

Besonders bevorzugt ist das Ubichinon der Formel (UBI-I) mit n = 10, auch bekannt als Coenzym Q10.

Erfindungsgemäß besonders bevorzugte Zusammensetzungen sind **dadurch gekennzeichnet, dass** sie mindestens ein Ubichinon, Ubichinol oder ein Derivat hiervon in einer Gesamtmenge von 0,0001 - 1 Gew.-%, bevorzugt 0,001 - 0,5 Gew.-% und besonders bevorzugt 0,005 - 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen mindestens eine natürliche Betainverbindung. Die Herkunft der eingesetzten natürlichen Betainverbindung kann erfindungsgemäß durchaus synthetisch sein. Erfindungsgemäß bevorzugte natürliche Betainverbindungen sind natürlich vorkommende Verbindungen mit der Atomgruppierung R₃N⁺-CH₂-X-COO⁻ gemäß IUPAC-Regel C-816.1. So genannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻), Carnitin (Me₃N⁺-CH₂-CHOH-CH₂-COO⁻), jeweils mit Me = Methyl, und Ergothionein = 2-Mercapto-N^{α}N^{α}N^{α}-trimethyl-L-histidinium-Betain der Formel (BETA-II)

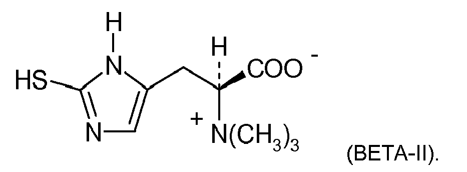

Weiterhin können auch Salze und/oder Ester der natürlichen Betainverbindungen erfindungsgemäß bevorzugt eingesetzt werden. Besonders bevorzugte derartige Derivate sind Carnitintartrat, Propionylcarnitin und insbesondere Acetylcarnitin. Beide Enantiomere (D-und L-Form) sind vorteilhaft im Sinne der vorliegenden Erfindung zu verwenden. Es kann auch von Vorteil sein, beliebige Enantiomerengemische, beispielsweise ein Racemat aus D-und L-Form, zu verwenden. Bevorzugte erfindungsgemäße Zusammensetzungen sind **dadurch gekennzeichnet, dass** sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte erfindungsgemäße Zusammensetzung, enthalten.

Weitere erfindungsgemäß bevorzugte Zusammensetzungen sind **dadurch gekennzeichnet, dass** sie mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.

Weitere erfindungsgemäß bevorzugte Zusammensetzungen sind **dadurch gekennzeichnet, dass** sie mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.

Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆₋₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/ oder Hydroxy-Gruppen enthalten, bevorzugt Substanzen mit den INCI-Bezeichnungen Dimethiconol, Amodimethicone oder Trimethylsilylamodimethicone.

Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte erfindungsgemäße Zusammensetzung.

Die erfindungsgemäßen kosmetischen oder dermatologischen Zusammensetzungen liegen in Form einer flüssigen, fließfähigen oder festen Öl-in-Wasser-Emulsion vor.

Weitere geeignete Zusatzstoffe sind Verdickungsmittel, z. B. anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolamin-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{®} NS, Simulgel^{®} EPG und Simulgel^{®} EG von SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemuleno^{®} und die Carbopol^{®}-Typen 954, 980, 1342 und ETD 2020 (ex Noveon).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Weitere bevorzugte Zusatzstoffe sind Lösemittel wie Ethanol, Isopropanol, Ethylenglykol, Propylenglycol, Propylenglykolmonoethylether, Glycerin und Diethylenglykol, Parfümöle, Substanzen zur Einstellung des pH-Werts, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

In einer weiteren bevorzugten Ausführungsform liegen die erfindungsgemäßen Zusammensetzungen in einem Sprühspender oder Pumpspender verpackt vor.

Gegenstand der Erfindung ist ferner die nicht-therapeutische, kosmetische Verwendung der erfindungsgemäßen vorstehend definierten Zusammensetzungen zum Schutz von Haut und Haar gegen die schädlichen Wirkungen von UV-Strahlung, insbesondere UV-A-und UV-B-Strahlung, insbesondere Sonnenstrahlung.

Bezüglich weiterer bevorzugter Ausführungsformen der erfindungsgemäßen Verwendungen gilt mutatis mutandis das zu den erfindungsgemäßen Zusammensetzungen Gesagte.

Ein weiterer Gegenstand der vorliegenden Anmeldung ist ein nicht-therapeutisches, kosmetisches Verfahren zum Schutz von Haut und Haar gegen die schädlichen Wirkungen von UV-Strahlung, insbesondere UV-A-und UV-B-Strahlung, insbesondere Sonnenstrahlung, bei dem eine erfindungsgemäße oder erfindungsgemäß bevorzugte Zusammensetzung gemäß einem der Ansprüche 1, 2, 3, 4, 5, 6, 7, 8, 9, 10, 11 oder 12 in einer wirksamen Menge auf die Haut aufgetragen wird.

Bezüglich weiterer bevorzugter Ausführungsformen des erfindungsgemäßen Verfahrens gilt mutatis mutandis das zu den erfindungsgemäßen Zusammensetzungen Gesagte.

Die nachfolgenden Beispiele sollen den Gegenstand der vorliegenden Anmeldung verdeutlichen, ohne ihn hierauf einzuschränken. Die Zahlenwerte in den Beispielen bedeuten Gewichtsprozente, bezogen auf das Gesamtgewicht der jeweiligen Zusammensetzungen.

### Beispiel 4: Neo Heliopan Hydro wurde in AMP gelöst; der pH wurde mit NaOH auf 7,3 eingestellt.

**Liste der verwendeten Rohstoffe**

| | | |
|---|---|---|
| Handelsname | INCI | Hersteller |
| Emulgade PL 68/50 | Cetearyl Glucoside, Cetearyl Alcohol | Cognis |
| Controx KS | Tocopherol, Hydrogenated Palm Glycerides Citrate | Cognis |
| Parsol 1789 | Butyl Methoxydibenzoylmethane | DSM |
| Parsol SLX | Polysilicone-15 | DSM |
| Tego Carbomer 140 | Carbomer | Goldschmidt |
| Neo Heliopan Hydro | Phenylbenzimidazol sulfonic acid | Symrise |
| Cetiol B | DIBUTYL ADIPATE | Cognis |
| Cetiol Sensoft | Propylheptyl Caprylate (2-Propylheptyl octanoate) | Cognis |
| Uvinul T 150 | ETHYLHEXYL TRIAZONE | BASF |
| AMP | Aminomethyl propanol (2-Amino-2-methylpropanol) | |
| Sunspheres | Styrene/Acrylates Copolymer | Rohm & Haas |
| Tego Carbomer 140 | Carbomer | Evonik |
| NTA-Na₃, 40% | Trisodium NTA (Nitrilotriessigsäuretrinatrium-Salz) | |
| Unimer U-151 | VP/Hexadecene Copolymer | Induchem |

**Testrezepturen (Öl-in-Wasser-Emulsionen) [alle Mengenangaben in Gew.-%]**

| | Nr.1 | Nr. 2 | Nr. 3 | Nr. 4 | Nr. 5 | Nr. 6 |
|---|---|---|---|---|---|---|
| Emulgade PL 68/50 | 8 | 6 | 6 | 3 | 3 | 3 |
| Cetiol B | 5 | 5 | 5 | 5 | 5 | 5 |
| Caprylic/ Capric Triglycerid | 3,5 | 3,5 | 3,5 | 3,5 | 3,5 | 3,5 |
| Distelöl | 2 | 2 | 2 | 2 | 2 | 2 |
| Behenylalkohol | 3 | 3 | 3 | 3 | 3 | 3 |
| Cetiol Sensoft | 0,8 | 1 | 1 | 1 | 1 | 1 |
| Tocopherylacetat | 0,5 | 0,5 | 0,5 | 0,5 | 0,5 | 0,5 |
| Controx KS | 0,05 | 0,05 | 0,05 | 0,05 | 0,05 | 0,05 |
| Bienenwachs | 1 | 0,2 | 0,2 | 0,2 | 0,2 | 0,2 |
| Parsol 1789 | 4 | 4 | 4 | 3 | 4 | 4 |
| Uvinul T 150 | 4 | 4 | 4 | - | 4 | 4 |
| Parsol SLX | - | - | - | 3 | - | - |
| Propylparaben | 0,2 | 0,2 | 0,2 | 0,2 | 0,2 | 0,2 |
| Hexandiol-1,6 | 5 | 5 | 5 | 5 | 5 | 5 |
| Glycerin, 86% | 4,5 | 4,5 | 4,5 | 4,5 | 4,5 | 4,5 |
| Sorbit, 70% | 2 | 2 | 2 | 2 | 2 | 2 |
| Sunspheres | 3 | 3 | - | - | 3 | - |
| Methylparaben | 0,2 | 0,2 | 0,2 | 0,2 | 0,2 | 0,2 |
| Tego Carbomer 140 | 0,5 | 0,5 | 0,5 | 0,5 | 0,5 | 0,5 |
| NTA-Na₃, 40% | 0,1 | 0,1 | 0,1 | 0,1 | 0,1 | 0,1 |
| Talkum | 1,3 | 1,3 | - | - | - | - |
| Cosmedia Silc | 2 | 2 | 2 | 2 | 2 | 2 |
| Unimer U-151 | - | - | 3 | - | - | 3 |
| AMP | - | - | - | 0,99 | - | - |
| Neo Heliopan Hydro | - | - | - | 3 | - | - |
| NaOH (fest) | - | - | - | 0,2 | - | - |
| Wasser | 49,35 | 51,95 | 53,25 | 57,06 | 56,25 | 56,25 |
| | | | | | | |
| pH | | | | 7,3 | - | - |
| | | | | | | |
| SPF direkt nach Herstellung | nicht eingelagert, zu feste Konsistenz | 22,0 | 29,7 | 44,7 | 18,5 | instabile Emulsion, stabil mit 6 Gew.-% Emulgade PL 68/50 |
| SPF nach 4 Wochen | | | 18,2 | 53,1 | | |
| SPF nach 8 Wochen | | | | 46,6 | | |
| Kristallisation des Neo Heliopan Hydro direkt nach Herstellung | | | | Keine Nadeln | | |
| Nach 4 Wochen | | | | keine Nadeln | | |
| Nach 8 Wochen | | | | keine Nadeln | | |

## Patentansprüche

1. Kosmetische Öl-in-Wasser-Emulsion, enthaltend
a) mindestens einen linearen, gesättigten primären Alkohol mit 14 - 30 Kohlenstoffatomen,
b) mindestens ein Alkylglycosid mit einem linearen oder verzweigten Alkyl- oder Alkenylrest mit 14 - 30 Kohlenstoffatomen und einem Mono-, Di- oder Triglycosidrest,
c) Polysilicone-15,
d) mindestens einen wasserlöslichen UV-Filter, umfassend ein Derivat der Benzimidazolsulfonsäure,
**gekennzeichnet durch** einen pH-Wert bei 20°C von 6,0 - 8, bevorzugt 6,5 - 7,5, besonders bevorzugt 6,8 bis 7,3.

2. Kosmetische Zusammensetzung gemäß Anspruch 1, **dadurch gekennzeichnet, dass** der mindestens eine wasserlösliche organische UV-Filter ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren, bevorzugt den entsprechenden Natrium-, Kalium- oder Triethanolamin-Salzen, insbesondere aus Phenylbenzimidazolsulfonsäure und dem Phenylen-1,4-bis-(2-benzimidazyl)-3,3'-5,5'-tetrasulfonsäure-bis-natriumsalz.

3. Kosmetische Zusammensetzung gemäß Anspruch 1 oder 2, **dadurch gekennzeichnet, dass** der mindestens eine wasserlösliche organische UV-Filter ausgewählt ist aus den Salzen von Phenylen-1,4-bis-(2-benzimidazyl)-3,3'-5,5'-tetrasulfonsäure oder 2-Phenylbenzimidazol-5-sulfonsäure mit Amino-C₁-C₆-Alkanolen und/oder Amino-C₁-C₆-Alkandiolen, insbesondere mit 2-Amino-2-methylpropan-1-ol, 2-Amino-2-methylpropan-1,3-diol, 2-Aminopropan-1-ol, 3-Aminopropan-1-ol, 1-Aminopropan-2-ol (MIPA) oder 2-Amino-2-(hydroxymethyl)propan-1,3-diol (TRIS), besonders bevorzugt mit 2-Amino-2-methylpropan-1-ol oder mit 2-Amino-2-methylpropan-1,3-diol.

4. Kosmetische Zusammensetzung gemäß Anspruch 1, 2 oder 3, **dadurch gekennzeichnet, dass** basische Aminosäuren, wie Lysin, Arginin und Histidin, in einer Gesamtmenge von 0 bis maximal 0,1 Gew.-%, bezogen auf die gesamte Zusammensetzung, enthalten sind.

5. Kosmetische Zusammensetzung gemäß Anspruch 1, 2, 3 oder 4, **dadurch gekennzeichnet, dass** der mindestens eine öllösliche UV-Filter eine Kombination aus mindestens einem Alkyl- und/oder Alkoxy-substituierten Dibenzoylmethanderivat und Polysilicone-15 umfasst.

6. Kosmetische Zusammensetzung gemäß Anspruch 5, **dadurch gekennzeichnet, dass** das mindestens eine Alkyl- und/oder Alkoxy-substituiertes Dibenzoylmethanderivat ausgewählt ist aus 4-(tert.-Butyl)-4'-methoxydibenzoylmethan.

7. Kosmetische Zusammensetzung gemäß Anspruch 1, 2, 3, 4, 5 oder 6, **dadurch gekennzeichnet, dass** a) 4-(tert.-Butyl)-4'-methoxydibenzoylmethan, b) Polysilicone-15 und c) 2-Phenylbenzimidazol-5-sulfonsäure(salz) enthalten sind.

8. Kosmetische Zusammensetzung gemäß Anspruch 7, **dadurch gekennzeichnet, dass** die UV-Filter a), b) und c) in einem Gewichtsverhältnis zueinander von a) : b) : c) wie (0,8 - 1,5) : (0,8 - 1,5) : (0,8- 1,5) enthalten sind.

9. Kosmetische Zusammensetzung gemäß Anspruch 7 oder 8, **dadurch gekennzeichnet, dass** die UV-Filter a), b) und c) in einem Gewichtsverhältnis zueinander von a) : b) : c) wie (0,9 - 1,2) : (0,9- 1,2) : (0,9- 1,2), bevorzugt (1 - 1,1) : (1 - 1,1) : (1 - 1,1), enthalten sind.

10. Kosmetische Zusammensetzung gemäß Anspruch 1, 2, 3, 4, 5, 6, 7, 8 oder 9, **dadurch gekennzeichnet, dass** ein Gemisch aus mindestens einem linearen, gesättigten primären Alkohol mit 14 - 30 Kohlenstoffatomen und mindestens einem Alkylglycosid mit einem linearen oder verzweigten Alkyl- oder Alkenylrest mit 14 - 30 Kohlenstoffatomen und einem Mono-, Di- oder Triglycosidrest in einer Gesamtmenge von 0,5 Gew.-% bis 8 Gew.-%, besonders bevorzugt 1 bis 6 Gew.-%, insbesondere 2,5 - 4,5 Gew.-% und außerordentlich bevorzugt 3 - 4 Gew.-%, enthalten ist.

11. Kosmetische Zusammensetzung gemäß Anspruch 1, 2, 3, 4, 5, 6, 7, 8, 9 oder 10, **dadurch gekennzeichnet, dass** sie frei ist von UV-Filtersubstanzen auf Basis von s-Triazinderivaten, die das nachfolgende Strukturmotiv 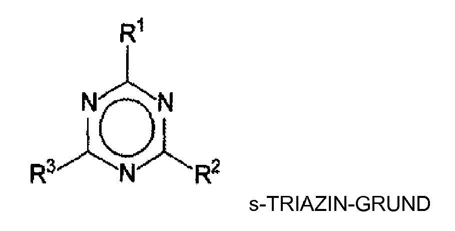 aufweisen.

12. Kosmetische Zusammensetzung gemäß Anspruch 1, 2, 3, 4, 5, 6, 7, 8, 9, 10 oder 11, **dadurch gekennzeichnet, dass** die mindestens eine Booster-Substanz e) für den Lichtschutzfaktor enthalten ist, die bevorzugt ausgewählt ist aus mindestens einer der folgenden vier Gruppen i) bis iv):
i. Dicarbonsäureester von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat,
ii. 2-Propylheptyloctanoat,
iii. mindestens einem Natriumacrylat-Homopolymer mit einem mittleren Molgewicht von 2500000 bis 3500000 Dalton, dessen 1 Gew.-%-ige wässrige Lösung einen pH-Wert von 5,5 - 7,5 aufweist,
iv. Siliciumdioxid-Partikel, die nicht organisch oder anorganisch modifiziert sind und keine organische oder anorganische Beschichtung aufweisen und zu mehr als 80 Gew.-% sphärische Partikel mit einem zahlenmittleren Partikeldurchmesser im Bereich von 2 - 15 µm enthalten.

13. Nicht-therapeutische, kosmetische Verwendung einer Zusammensetzung gemäß Anspruch 1, 2, 3, 4, 5, 6, 7, 8, 9, 10, 11 oder 12 zum Schutz von Haut und Haar gegen die schädlichen Wirkungen von UV-Strahlung, insbesondere UV-A-und UV-B-Strahlung, insbesondere Sonnenstrahlung.

14. Nicht-therapeutisches, kosmetisches Verfahren zum Schutz von Haut und Haar gegen die schädlichen Wirkungen von UV-Strahlung, insbesondere UV-A-und UV-B-Strahlung, insbesondere Sonnenstrahlung, **dadurch gekennzeichnet, dass** eine Zusammensetzung gemäß einem der Ansprüche 1, 2, 3, 4, 5, 6, 7, 8, 9, 10, 11 oder 12 in einer wirksamen Menge auf die Haut aufgetragen wird.
